# EUROPEAN PATENT APPLICATION

(11) **EP 4 289 435 A1**
(43) Date of publication of application: **13.12.2023**
(21) Application number: 22177540.6
(22) Date of filing: 07.06.2022
(51) Int. Cl.: A61K 31/7076, A61L 2/00, A61P 31/04, A61P 43/00

(54) **N-ALKYL-2-SUBSTITUTED ATP ANALOGUES FOR USE AS ANTIBACTERIAL AGENT**

(71) Applicant: Université de Liège, 4000 Liège (BE)
(72) Inventor: Oury, Cécile, 4140 Sprimont (BE); Jacques, Nicolas, 6600 Bastogne (BE); Lancellotti, Patrizio, 4140 Louveigné (BE)
(74) Representative: De Clercq & Partners

(57) **Abstract**

The present invention relates to a compound of formula I, a tautomer, an enantiomer or a diastereomer thereof, 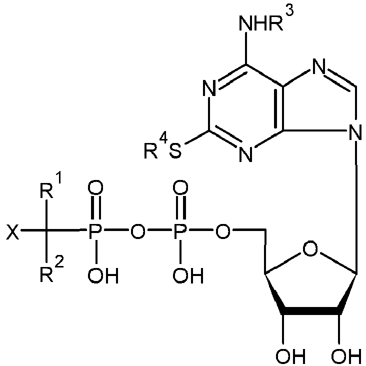 wherein R¹, R², R³ and X have the same meaning as that defined in the claims and the description. The present invention also relates to uses of such compounds as inhibitors of bacterial adhesion and biofilm formation on a surface, methods ex-vivo for preventing bacterial growth in biofilm formation and the use of such compounds in diagnosing or prognosing bacterial infections.

## Description

### Field of the invention

The present invention relates to newly discovered antibacterial properties of N-alkyl-2-substituted ATP analogues, used clinically for another indication. The newly discovered antibacterial properties of the said analogues can be applied in the prevention or treatment of bacterial infections in a subject; in addition, the N-alkyl-2-substituted ATP analogues may be used ex-vivo or in vitro and to inhibit bacterial adhesion and biofilm formation on surfaces.

### Background of the invention

Cangrelor is a synthetic analogue of adenosine triphosphate (ATP) belonging to the N-alkyl-2-substituted ATP analogues and a potent antagonist of the P2Y₁₂ receptor, a G-protein coupled purinergic receptor which is an important component of platelet activation. Cangrelor is represented by the following formula II: 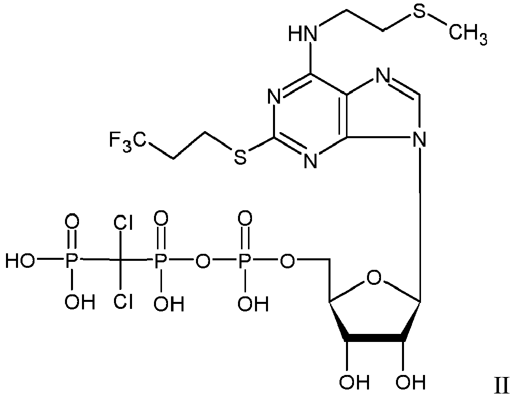 or by its sodium salt represented by formula III:

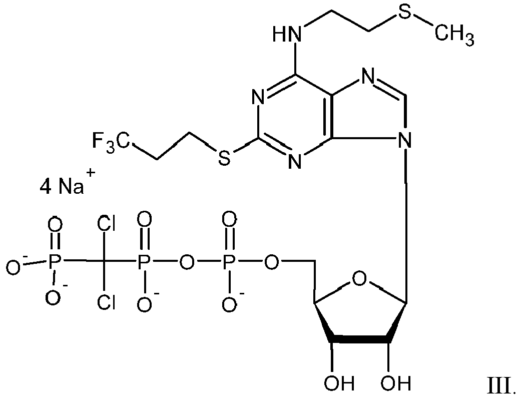

Cangrelor is the only intravenous platelet P2Y₁₂ inhibitor currently available for clinical use. It provides prompt, potent and reliable antiplatelet effects. Such pharmacological properties allow to overcome limitations of oral P2Y₁₂ inhibitors characterized by inevitable delay in their onset of action, which is enhanced in high risk short-term settings in which gastrointestinal absorption is further compromised (De Luca et al., J Am Heart Assoc. 2021;10:e022125).

Antibiotics are used to prevent or treat bacterial infection. However, due to the ability of bacteria to rapidly develop resistance mechanisms along with antibiotics misuse, we are currently facing a global spread of resistant bacteria worldwide. Currently available antibiotics are becoming increasingly ineffective, and there are limited treatment options for common infections such as urinary tract infection, sepsis, sexually transmitted infections, and some forms of diarrhea. This phenomenon also threatens the safety of surgery, chemotherapy or other treatments.

The World Health Organization predicts that antibiotic resistance could result in 10 million deaths annually by 2050. A study in The Lancet (2022) indicates that resistant bacteria directly accounted for 1.27 million deaths in 2019 (Antimicrobial Resistance Collaborators. Global burden of bacterial antimicrobial resistance in 2019: a systematic analysis. Lancet. 2022 Feb 12;399(10325):629-655. doi: 10.1016/S0140-6736(21)02724-0).

Infections caused by Gram-positive bacteria represent a major public health burden, not just in terms of morbidity and mortality, but also in terms of increased expenditure on patient management and implementation of infection control measures. More particularly, *Staphylococcus aureus* and *enterococci* are established pathogens in the hospital environment, and their frequent multidrug resistance complicates therapy.

*Staphylococcus aureus* is an important highly virulent pathogen responsible for a broad range of clinical manifestations ranging from relatively benign skin infections to life-threatening conditions such as endocarditis and osteomyelitis. It is also a commensal bacterium (colonizing approximately 30 percent of the human population).

Two major shifts in *S. aureus* epidemiology have occurred since the 1990s: an epidemic of community-associated skin and soft tissue infections (largely driven by specific methicillin-resistant *S. aureus* [MRSA] strains), and an increase in the number of healthcare-associated infections (especially infective endocarditis and prosthetic device infections). Patients with MRSA infections are 64% more likely to die than people with drug-sensitive infections.

There is therefore an urgent need in the art for new antibacterial treatment alternatives.

*S. aureus* pathogenicity depends on the expression of a wide array of virulence factors. Among these factors, toxins are key virulence determinants with cytotoxic properties enabling the bacteria to escape the host immune system. The carotenoid pigment staphyloxanthin helps *S. aureus* to resist reactive oxygen species-dependent killing by host neutrophils. Bacterial virulence is also dictated by the ability of the bacteria to adhere to host tissues or surfaces via diverse mechanisms, which is the initial step of bacterial colonization, biofilm formation and infection.

Antibacterial treatment alternatives to classical antibiotics involve agents able to inhibit bacterial virulence factors, so-called anti-virulence agents. Clinical trials are currently testing such anti-virulence agents for the prevention or treatment of multi-resistant bacterial infections (Imperi F, Chen W, Smani Y. Front Microbiol. 2021;12). These anti-virulence agents represent valuable alternatives to antibiotics (Ogawara H. J Antibiot (Tokyo). 2021;74:24-41). Indeed, in contrast to antibiotics, these drugs would not impose a high selective pressure on bacteria, therefore limiting the development of resistance and dissemination of virulence genes.

In 2017, C. Oury et al. described in EP3509598B1 a new use of triazolo(4,5-D)pyrimidine derivatives for prevention and treatment of bacterial infection, but triazolo(4,5-D)pyrimidine derivatives such as Ticagrelor are generally not soluble in an aqueous environment or medium.

### Summary of the invention

The present invention is based on the unexpected finding that at least one of the above mentioned objectives can be attained by N-alkyl-2-substituted ATP analogues, used clinically for another indication.

The present invention provides N-alkyl-2-substituted ATP analogues that have antibacterial properties. The analogues are capable of inhibiting the haemolytic activity of clinically relevant strains of *Staphylococcus aureus,* such as a clinical isolate from a patient with infective endocarditis, or a methicillin-resistant strain. In addition, the present inventors have observed that the analogues of the invention can inhibit the production of the major bacterial pigment, staphyloxantin. Since staphyloxanthin has antioxidant activity that helps the bacteria evade killing by reactive oxygen species produced by phagocytic innate immune cells, these data further indicate that the analogues of the invention are able to reduce the virulence of *Staphylococcus aureus.* Accordingly, the compounds of the invention are suitable for use as antibacterial agents, more particularly in the treatment and/or prevention of diseases caused by bacterial infections. By antibacterial agent is here also meant anti-virulence agent or anti-infectious agent.

In contrast to triazolo(4,5-D)pyrimidine derivatives such as Ticagrelor, the compounds of the invention are highly soluble in an aqueous environment or medium. This property would enable administration via intravenous route, leading to a very rapid onset of action, which could particularly be important in high-risk short-term settings in which gastrointestinal absorption is compromised. The water soluble property could also be advantageous for use of the compounds of the invention in water-based compositions or coatings, such as hydrogels or nanogels or the like.

A first aspect of the present invention provides a compound of formula I, a tautomer, an enantiomer or a diastereomer thereof, 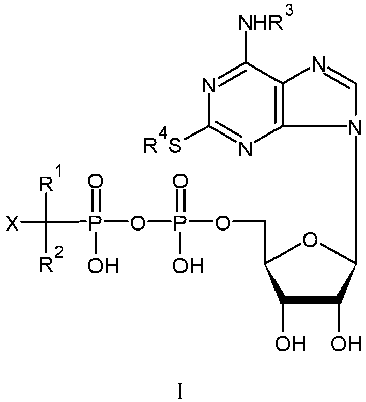 wherein,
R¹ and R² are each independently hydrogen or halogen;
R³ and R⁴ are each independently phenyl, or C₁₋₆alkyl; wherein each phenyl or C₁₋₆alkyl can be substituted or unsubstituted with one or more substituents each independently selected from the group consisting of OR⁵, C₁₋₆alkylthio, NR⁶R⁷, phenyl, COOR⁸ or halogen;
R⁵, R⁶, R⁷ and R⁸ are each independently hydrogen or C₁₋₆alkyl,
X is an acidic moiety;
or a salt or a solvate thereof or a solvate of such a salt;
for use in the prevention or treatment of a bacterial infection in a subject.

The present invention is also related to a pharmaceutical composition comprising the compound of formula (I) for use in the prevention or treatment of a bacterial infection in a subject.

A further related aspect of the present invention provides the use of a compound of formula I as described herein as an antibacterial agent, more particularly as an inhibitor of bacterial adhesion and biofilm formation on a surface.

A further related aspect of the present invention provides a method ex-vivo for preventing bacterial growth in biofilm formation comprising applying or grafting on a surface an effective amount of a compound of formula I as described herein.

Yet a further related aspect of the present invention provides a compound of formula I as described herein for use in diagnosing or prognosing bacterial infection. Such a diagnosing or prognosing may be for example with a detectable marker or with any suitable method.

### Brief description of the figures

The following description of the figures of specific embodiments of the invention is merely exemplary in nature and is not intended to limit the present teachings, their application or uses.
Figure 1 represents a graph plotting the percentage of haemolytic activity of *S. aureus* isolated from a patient with infective endocarditis (IE strain), against either a control or tetrasodium cangrelor in 0.5 µg/ml. The haemolytic activity obtained for the control was arbitrarily set to 100%.
Figure 2 represents a graph plotting the percentage of haemolytic activity of a methicillin-resostamt JE2 (USA300) *S. aureus* strain, against either a control or tetrasodium cangrelor in 0.5 µg/ml. The haemolytic activity obtained for the control was arbitrarily set to 100%.
Figure 3 represents a graph plotting the percentage of pigment production (staphyloxantin) by the IE strain of *S. aureus,* against either a control or tetrasodium cangrelor in 0.5 µg/ml. The pigment production obtained for the control was arbitrarily set to 100%.

### Detailed description of the invention

Before the present invention is described, it is to be understood that this invention is not limited to particular processes, methods, and compounds described, as such processes, methods, and compounds may, of course, vary. It is also to be understood that the terminology used herein is not intended to be limiting, since the scope of the present invention will be limited only by the appended claims.

When describing the compounds and processes of the invention, the terms used are to be construed in accordance with the following definitions, unless a context dictates otherwise.

As used in the specification and the appended claims, the singular forms "a", "an," and "the" include both singular and plural referents unless the context clearly dictates otherwise. By way of example, "a compound" means one compound or more than one compound.

The terms "comprising", "comprises" and "comprised of" as used herein are synonymous with "including", "includes" or "containing", "contains", and are inclusive or open-ended and do not exclude additional, non-recited members, elements or method steps. The terms "comprising", "comprises" and "comprised of" also include the term "consisting of".

The term "about" as used herein when referring to a measurable value such as a parameter, an amount, a temporal duration, and the like, is meant to encompass variations of +/-10% or less, preferably +/-5% or less, more preferably +/-1% or less, and still more preferably +/-0.1% or less of and from the specified value, insofar such variations are appropriate to perform in the disclosed invention. It is to be understood that the value to which the modifier "about" refers is itself also specifically, and preferably, disclosed.

As used herein, the term "and/or," when used in a list of two or more items, means that any one of the listed items can be employed by itself or any combination of two or more of the listed items can be employed. For example, if a list is described as comprising group A, B, and/or C, the list can comprise A alone; B alone; C alone; A and B in combination; A and C in combination, B and C in combination; or A, B, and C in combination.

The recitation of numerical ranges by endpoints includes all integer numbers and, where appropriate, fractions subsumed within that range (e.g. 1 to 5 can include 1, 2, 3, 4 when referring to, for example, a number of elements, and can also include 1.5, 2, 2.75 and 3.80, when referring to, for example, measurements). The recitation of end points also includes the end point values themselves (e.g. from 1.0 to 5.0 includes both 1.0 and 5.0). Any numerical range recited herein is intended to include all sub-ranges subsumed therein.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiments but may. Furthermore, the particular features, structures or characteristics may be combined in any suitable manner, as would be apparent to a person skilled in the art from this disclosure, in one or more embodiments. Furthermore, while some embodiments described herein include some but not other features included in other embodiments combinations of features of different embodiments are meant to be within the scope of the invention, and form different embodiments as would be understood by those in the art. For example, in the following claims, any of the claimed embodiments can be used in any combination. Unless otherwise defined, all terms used in disclosing the invention, including technical and scientific terms, have the meaning as commonly understood by one of ordinary skill in the art to which this invention belongs. By means of further guidance, definitions for the terms used in the description are included to better appreciate the teaching of the present invention.

When describing the present invention, the terms used are to be construed in accordance with the following definitions, unless a context dictates otherwise.

The terms described above and others used in the specification are well understood to those in the art.

Whenever the term "substituted" is used herein, it is meant to indicate that one or more hydrogen atoms on the atom indicated in the expression using "substituted" is replaced with a selection from the indicated group, provided that the indicated atom's normal valence is not exceeded, and that the substitution results in a chemically stable compound, i.e. a compound that is sufficiently robust to survive isolation from a reaction mixture.

Where groups can be substituted, such groups may be substituted with one or more, and preferably one, two or three substituents. Preferred substituents may be selected from but not limited to, for example, the group comprising halo, hydroxyl, alkyl, alkoxy, trifluoromethyl, trifluoromethoxy, cycloalkyl, aryl, arylalkyl, heterocyclyl, heteroaryl, cyano, amino, nitro, carboxyl, and mono- or dialkylamino.

The term "halo" or "halogen" as a group or part of a group is generic for fluoro, chloro, bromo, iodo.

The term "alkyl", as a group or part of a group, refers to a hydrocarbyl group of formula -CₙH₂ₙ₊₁ wherein n is a number greater than or equal to 1. Alkyl groups may be linear or branched and may be substituted as indicated herein. Generally, alkyl groups of this invention comprise from 1 to 6 carbon atoms, preferably from 1 to 5 carbon atoms, preferably from 1 to 4 carbon atoms, more preferably from 1 to 3 carbon atoms, still more preferably 1 to 2 carbon atoms. When a subscript is used herein following a carbon atom, the subscript refers to the number of carbon atoms that the named group may contain. For example, "C₁₋₆alkyl" includes all linear or branched alkyl groups with between 1 and 6 carbon atoms, and thus includes methyl, ethyl, n-propyl, i-propyl, butyl and its isomers (e.g. n-butyl, i-butyl and t-butyl); pentyl and its isomers, hexyl and its isomers. For example, "C₁₋₅alkyl" includes all includes all linear or branched alkyl groups with between 1 and 5 carbon atoms, and thus includes methyl, ethyl, n-propyl, i-propyl, butyl and its isomers (e.g. n-butyl, i-butyl and t-butyl); pentyl and its isomers. For example, "C₁₋₄alkyl" includes all linear or branched alkyl groups with between 1 and 4 carbon atoms, and thus includes methyl, ethyl, n-propyl, i-propyl, butyl and its isomers (e.g. n-butyl, i-butyl and t-butyl). For example "C₁₋₃alkyl" includes all linear or branched alkyl groups with between 1 and 3 carbon atoms, and thus includes methyl, ethyl, n-propyl, i-propyl.

The term "alkoxy" or "alkyloxy", as a group or part of a group, refers to a group having the formula -OR^{b} wherein R^{b} is alkyl as defined herein above. Non-limiting examples of suitable alkoxy include methoxy, ethoxy, propoxy, isopropoxy, butoxy, isobutoxy, sec-butoxy, tert-butoxy, pentyloxy and hexyloxy.

The term "C₁₋₆alkylthio ", as a group or part of a group, refers to a group having the formula -SR^{b} wherein R^{b} is alkyl as defined herein above. Non-limiting examples of suitable C₁₋₆alkylthio include methylthio, ethylthio, propylthio, isopropylthio, butylthio, isobutylthio, sec-butylthio, tert-butylthio, pentylthio and hexylthio.

The term "mono- or di-alkylamino", as a group or part of a group, refers to a group of formula -N(R^{o})(R^{p}) wherein R° and R^{p} are each independently selected from hydrogen, or alkyl, wherein at least one of R° or R^{p} is alkyl. Thus, alkylamino include mono-alkyl amino group (e.g. mono-C₁₋₆alkylamino group such as methylamino and ethylamino), and di-alkylamino group (e.g. di-C₁₋₆alkylamino group such as dimethylamino and diethylamino). Non-limiting examples of suitable mono- or di-alkylamino groups include *n*-propylamino, isopropylamino, *n*-butylamino, i-butylamino, sec-butylamino, t-butylamino, pentylamino, *n*-hexylamino, di-*n*-propylamino, di-i-propylamino, ethylmethylamino, methyl-*n*-propylamino, methyl-i-propylamino, *n-*butylmethylamino, *i*-butylmethylamino, *t*-butylmethylamino, ethyl-*n*-propylamino, ethyl-i-propylamino, *n*-butylethylamino, i-butylethylamino, *t*-butylethylamino, di-*n*-butylamino, di-i-butylamino, methylpentylamino, methylhexylamino, ethylpentylamino, ethylhexylamino, propylpentylamino, propylhexylamino, and the like.

A structural isomer is a type of isomer in which molecules with the same molecular formula have different bonding patterns and atomic organization. Where structural isomers are interconvertible via a low energy barrier, tautomeric isomerism ('tautomerism') can occur. This can take the form of proton tautomerism in compounds of the invention containing, for example, an imino, keto, or oxime group, or so-called valence tautomerism in compounds which contain an aromatic moiety. The compounds of formula I may exhibit tautomerism, e.g. imine-enamine tautomerism at the 6-position of adenine. The compounds also contain one or more asymmetric carbon atoms and therefore exhibit optical and/or diastereoisomerism.

The present invention includes all possible stereoisomers compounds of formula I and any subgroup thereof. When a compound is desired as a single enantiomer, such may be obtained by stereospecific synthesis, by resolution of the final product or any convenient intermediate, or by chiral chromatographic methods as each are known in the art. Resolution of the final product, an intermediate, or a starting material may be effected by any suitable method known in the art. See, for example, Stereochemistry of Organic Compounds by E. L. Eliel, S. H. Wilen, and L. N. Mander (Wiley- Interscience, 1994), incorporated by reference with regard to stereochemistry.

Preferred statements (features) and embodiments of the compounds and processes of this invention are now set forth. Each statement and embodiment of the invention so defined may be combined with any other statement and/or embodiments unless clearly indicated to the contrary. In particular, any feature indicated as being preferred or advantageous may be combined with any other feature or features indicated as being preferred or advantageous.

A first aspect of the present invention provides a compound of formula I, a tautomer, an enantiomer or a diastereomer thereof, 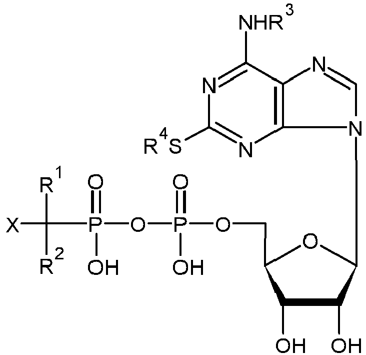 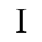 wherein,
R¹ and R² are each independently hydrogen or halogen;
R³ and R⁴ are each independently phenyl, or C₁₋₆alkyl; wherein each phenyl or C₁₋₆alkyl can be substituted or unsubstituted with one or more substituents each independently selected from the group consisting of OR⁵, C₁₋₆alkylthio, NR⁶R⁷, phenyl, COOR⁸ or halogen;
R⁵, R⁶, R⁷ and R⁸ are each independently hydrogen or C₁₋₆alkyl,
X is an acidic moiety;
or a salt or solvate thereof, or a solvate of such a salt;
for use in the prevention or treatment of a bacterial infection in a subject.

The present invention is also related to a pharmaceutical composition comprising the compound of formula I for use in the prevention or treatment of a bacterial infection in a subject.

The pharmaceutical compositions may include, in addition to the compound of formula I, auxiliary substances, preservatives, solvents and/or viscosity modulating agents. By solvent, one means for example water, saline solution or any other physiological solution, ethanol, glycerol, oil such as vegetable oil or a mixture thereof. By viscosity modulating agent one means for example carboxymethylcellulose.

A further related aspect of the present invention provides the use of a compound of formula I as described herein as inhibitor of bacterial adhesion and biofilm formation on a surface.

A further aspect of the present invention provides a method ex-vivo for preventing bacterial growth in biofilm formation comprising applying or grafting on a surface an effective amount of a compound of formula I as described herein.

Yet a further related aspect of the present invention provides a compound of formula I as described herein for use in diagnosing or prognosing bacterial infection.

For example, when used in diagnosing or prognosing the compound of formula I may comprise a detectable marker.

The term "detectable marker" as used herein refers to any type of tag which is detectable and thus allows the determination of the presence of the compound of formula I. In particular embodiments, the marker is an isotope which allows the use of the compound of formula I as a radiotracer.

Acidic moieties which may represent X include Bronsted-Lowry acids, that is, moieties which act as proton donors. The acidic moiety may be mono- or poly-acidic. In some embodiments X is selected from the group consisting of -P(O)(OH)₂, -SO₃H or -CO₂H. In some embodiments X is - P(O)(OH)₂.

In some embodiments R⁴ is C₁₋₆alkyl, and wherein said C₁₋₆alkyl is substituted with one, two or three substituents. In some embodiments R⁴ is C₁₋₆alkyl, and wherein said C₁₋₆alkyl is substituted with one, two or three substituents independently selected from halo. In some embodiments R⁴ is C₁₋₆alkyl, and wherein said C₁₋₆alkyl is substituted with two or three substituents selected from halogen, wherein said two or three substituents are the same.

In some embodiments R³ is C₁₋₆alkyl, and wherein said C₁₋₆alkyl is substituted with one, two or three substituents. In some embodiments R³ is C₁₋₆alkyl, and wherein said C₁₋₆alkyl is substituted with one, two or three substituents independently selected from C₁₋₆alkylthio. In some embodiments R³ is C₁₋₆alkyl, and wherein said C₁₋₆alkyl is substituted with one substituent selected from C₁₋₆alkylthio. In some embodiments R³ is C₁₋₂alkyl, and wherein said C₁₋₂alkyl is substituted with one substituent selected from methylthio.

In some embodiments R¹ and R² are the same. In some embodiments R¹ and R² are the same and represent Cl.

The compounds of the invention may be in the form of salts as generally described below. Some preferred, but non-limiting examples of suitable organic and/or inorganic acids are as hydrochloric acid, hydrobromic acid, sulfuric acid, nitric acid, acetic acid and citric acid, as well as other pharmaceutically acceptable acids known per se (see Handbook of Pharmaceutical Salts: Properties, Selection, and Use by Stahl and Wermuth (Wiley-VCH, 2002), incorporated herein by reference).

When the compounds of the invention contain an acidic group as well as a basic group the compounds of the invention may also form internal salts, and such compounds are within the scope of the invention. When the compounds of the invention contain a hydrogen-donating heteroatom (e.g. NH), the invention also covers salts and/or isomers formed by transfer of said hydrogen atom to a basic group or atom within the molecule.

Salts of the compounds of formula I may be formed by reacting the free acid, or a salt thereof, or the free base, or a salt or derivative thereof, with one or more equivalents of the appropriate base or acid. The reaction may be carried out in a solvent or medium in which the salt is insoluble or in a solvent in which the salt is soluble, e.g. ethanol, tetrahydrofuran or diethyl ether, which may be removed in vacuo, or by freeze drying or other method known in the art. The reaction may also be a metathetical process or it may be carried out on an ion exchange resin.

Salts of the compounds of formula I include the acid addition and base salts thereof. Suitable acid addition salts are formed from acids which form non-toxic salts. Examples include the acetate, adipate, aspartate, benzoate, besylate, bicarbonate/carbonate, bisulfate/sulfate, borate, camsylate, citrate, cyclamate, edisylate, esylate, formate, fumarate, gluceptate, gluconate, glucuronate, hexafluorophosphate, hibenzate, hydrochloride/chloride, hydrobromide/bromide, hydroiodide/iodide, isethionate, lactate, malate, maleate, malonate, mesylate, methylsulphate, naphthylate, 2-napsylate, nicotinate, nitrate, orotate, oxalate, palmitate, pamoate, phosphate/hydrogen phosphate/dihydrogen phosphate, pyroglutamate, saccharate, stearate, succinate, tannate, tartrate, tosylate, trifluoroacetate and xinofoate salts. Suitable base salts are formed from bases which form non-toxic salts. Examples include the aluminium, arginine, benzathine, calcium, choline, diethylamine, diolamine, glycine, lysine, magnesium, meglumine, olamine, potassium, sodium, tromethamine and zinc salts. Hemisalts of acids and bases may also be formed, for example, hemisulphate and hemicalcium salts. For a review on suitable salts, see Handbook of Pharmaceutical Salts: Properties, Selection, and Use by Stahl and Wermuth (Wiley-VCH, 2002), incorporated herein by reference.

Salts of the compounds of formula I include alkali metal salts, e.g. sodium and potassium salts; alkaline earth metal salts, e.g. calcium and magnesium salts; salts of the Group III elements, e.g. aluminium salts; and ammonium salts. Salts with suitable organic bases, for example, salts with hydroxylamine; lower alkylamines, e.g. methylamine or ethylamine; with substituted lower alkylamines, e.g. hydroxy substituted alkylamines; or with monocyclic nitrogen heterocyclic compounds, e.g. piperidine or morpholine; and salts with amino acids, e.g. with arginine, lysine etc, or an N-alkyl derivative thereof; or with an aminosugar, e.g. N-methyl-D-glucamine or glucosamine. In some embodiments, the salts are pharmaceutically acceptable salts. Pharmaceutically acceptable salts constitute the non-toxic physiologically acceptable salts of the compounds of formula I described hereinabove.

In some embodiments the compound is a salt; preferably a sodium salt of a compound of formula I.

In some embodiments the compound of formula I is: 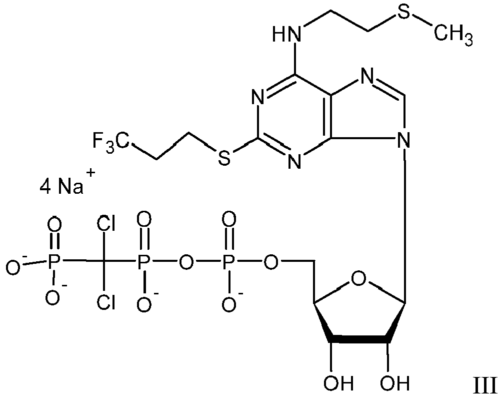 also referred to as tetrasodium cangrelor or cangrelor tetrasodium.

An aspect of the present invention provides the compound according to formula I as described herein, for use in the prevention or treatment of a bacterial infection in a subject. In some embodiments the bacterial infection is an infection caused by gram positive or gram negative bacteria.

Thus, the present invention also provides a method for the treatment of a bacterial infection in a subject in need thereof, said method comprising administering to the subject an effective amount of a compound according to formula I as described herein.

In some embodiments the subject is a mammal. In some embodiments the subject is a human. In some embodiments the subject is a human host. In particular embodiments, the infection is an infection of a bodily surface. Bodily surfaces include but are not limited to epidermis and mucous membranes. In some embodiments the surface is a bodily surface. In some embodiments the bodily surface is selected from an intact epidermal surface, a damaged epidermal surface or a mucosal surface.

Damaged epidermal surface may be skin which is blistered, burnt by fire, inflamed, pustulated, sunburnt, bitten, stung or otherwise wounded. Suitable examples of mucosal surfaces include the mucosa of the mouth (including tongue), nose, eyes, throat, oesophagus, stomach vagina and rectum.

In some embodiments the bacterial infection is an infection caused by gram positive bacteria. Examples of gram positive bacteria are: *Staphylococcus aureus, Staphylococcus epidermidis Bacillus anthracis, Corynebacterium diphtheriae, Enterococcus faecalis*, *Entereococcus faecium*, *Erysipelothrix rhusiopathiae, Listeria monocytogenes, Nocardia sp., Streptococcus pneumoniae, Streptococcus agalactiae.* In some embodiments the bacterial infection is an infection caused by *Staphylococcus*; preferably an infection caused by *Staphylococcus aureus*; preferably an infection caused by methicillin-resistant *Staphylococcus aureus.*

In some embodiments the bacterial infection is an infection caused by gram negative bacteria such as *Acinetobacter spp., such as Acinetobacter baumannii, Bordetella pertussis, Campylobacter spp.; Enterobacteriaceae such as Citrobacter spp., Enterobacter spp., Escherichia coli, Klebsiella spp., Salmonella spp., Serratia marcescens, Shigella spp., Yersinia spp.; Haemophilus influenza, Helocobacter pylorilegionella pneumophila, Neisseria spp., Pseudomonas aeruginosa, Vibrio cholera* and the like.

In some embodiments wherein the bacterial infection is caused by a gram negative bacteria, the compound of formula I as described herein is used together with a membrane penetrating agent. In some embodiments the membrane penetrating agent is selected from the group consisting of polymyxin B and polymyxin E.

A further related aspect of the present invention provides a use of a compound of formula I as described herein, as inhibitor of bacterial adhesion and biofilm formation on a surface.

By surface one means any type of surface such as rubber or plastic surface as for example surface made of polyethylene, polypropylene, polyurethane, polyvinyl chloride, polyvinylpyrrolidone, polytetrafluoroethylene, silicone or the like, or copolymers but also and preferably metallic surface such as stainless steel, silver, gold, titanium, metallic alloys, pyrolitic carbon, and the like. In some embodiments the surface is the surface of a biomaterial of a medical device. The present invention may also be used on bioabsorbable or biomaterial surface such as biological prosthesis or devices which are made of biological material such as for example porcine or bovine pericardium.

*Staphylococcus aureus* and coagulase-negative staphylococci (often *Staphylococcus epidermidis*) cause 65 to 75 percent of generator pocket infections and up to 89 percent of device-related endocarditis. Episodes arising within two weeks of implantation are more likely to be due to *S*. *aureus.*

Prosthetic valve endocarditis (PVE) is a serious infection with potentially fatal consequences. Bacteria can reach the valve prosthesis by direct contamination intraoperatively or via hematogenous spread during the initial days and weeks after surgery. The bacteria have direct access to the prosthesis-annulus interface and to perivalvular tissue along suture pathways because the valve sewing ring, cardiac annulus, and anchoring sutures are not endothelialized early after valve implantation. These structures are coated with host proteins, such as fibronectin and fibrinogen, to which some organisms can adhere and initiate infection.

The risk of developing prosthetic valve endocarditis (PVE) is greatest during the initial three months after surgery, remains high through the sixth month, and then falls gradually with an annual rate of approximately 0.4 percent from 12 months postoperatively onward. The percentage of patients developing PVE during the initial year after valve replacement ranges from 1 to 3 percent in studies with active follow-up; by five years, the cumulative percentage ranges from 3 to 6 percent.

The most frequently encountered pathogens in early PVE (within two months of implantation) are *S. aureus* and coagulase-negative staphylococci. The most frequently encountered pathogens in late PVE (two months after valve implantation) are streptococci and *S. aureus,* followed by coagulase-negative staphylococci and enterococci. The coagulase-negative staphylococci causing PVE during the initial year after surgery are almost exclusively *Staphylococcus epidermidis.* Between 84 and 87 percent of these organisms are methicillin resistant and thus resistant to all of the beta-lactam antibiotics.

It is known that PVE accounts for about 20 percent of all infective endocarditis. PVE is related to health care in about 30 percent of cases. *S. aureus* is the first causative pathogen, being responsible for more than 20 percent of PVE.

Periprosthetic joint infection (PJI) occurs in 1 to 2 percent of joint replacement surgeries and is a leading cause of arthroplasty failure. Biofilms play an important role in the pathogenesis of PJIs. Bacteria within biofilm become resistant to therapy; as a result, antibacterial therapy is often unsuccessful unless the biofilm is physically disrupted or removed by surgical debridement. The management of PJIs generally consists of both surgery and antibacterial therapy.

Accordingly, the use of the compounds of formula I according to the present invention is advantageous in the treatment, prevention and management of the above-described conditions by administering the compounds of formula I to a surface of a medical device.

A medical device includes, but is not limited to, any device, tool, instrument, implant, or the like, relating to medicine or the practice of human or veterinary medicine, or intended for use to heal or treat a disease or condition. A medical device may include all natural and synthetic materials and both fibrous and non-fibrous materials. For example, the materials may be comprised of a metal, plastic, paper, glass, ceramic, textile, rubber, polymer, composite material or any other material or combination of materials. Exemplary medical devices include, but are not limited to, any kind of catheter; cannulae; needles; stents of any size, shape, or placement; coils of any size, shape, or placement; contact lenses; IUDs; peristaltic pump chambers; endotracheal tubes; gastroenteric feeding tubes; arteriovenous shunts; condoms; oxygenator and kidney membranes; gloves; pacemaker leads; wound dressings; metallic pins, plates and screws; metallic artificial hips; artificial knees and the likes.

In some embodiments the surface is the surface of a cardiovascular device, or the surface of a catheter. In some embodiments the surface is the surface of a prosthetic heart valve, a pacemaker, a cardioverter-defibrillator, a cardiac ablation catheter, a cardiovascular angioplasty device, a ventricular assist device, or a catheter.

Examples of cardiovascular devices suitable for the present invention include: prosthetic heart valves, pacemakers, cardioverter-defibrillators, cardiac ablation catheters, cardiovascular angioplasty devices, ventricular assist devices (mechanical pumps) and the like.

As used herein the term "biomaterial" refers to a substance that has been engineered to interact with biological systems for a medical purpose. Biomaterials may be used in the following applications: joint replacements, bone plates, intraocular lenses, bone cement, artificial ligaments and tendons, dental implants, blood vessel prostheses, heart valves, skin repair devices (such as wound dressings), cochlear replacements, contact lenses, breast implants, drug delivery mechanisms, sustainable materials, vascular grafts, stents, nerve conduits, surgical sutures, clips and staples for wound closure, pins, screws for fracture stabilisation and surgical mesh and the like.

In particular embodiments, the use according to this aspect of the invention encompasses applying the compound of formula I as described herein to said surface. In particular embodiments, the application is ensured by spraying a composition comprising the compound of formula I on said surface.

The compound of formula I according to the invention may be applied to the surface of said biomaterial or medical device in the form of a coating.

As used herein "coating" is a covering that is applied to a surface. Coating of a surface may be achieved by any method known in the art, such as but not limited to spraying or by submerging the device (or a surface thereof) in a composition comprising the compound of formula I. Accordingly, the present invention also provides a composition comprising:
- a compound of formula I as described herein;
- a solubilizing vehicle.

Suitable solubilizing vehicles to prepare the compositions according to the present invention include, water, water-based solutions, alcohols, dimethyl sulfoxide and mixtures thereof. In some embodiments the solubilizing vehicle is a water-based solution. Suitable water-based solutions to prepare the compositions according to the present invention include culture medium (such as tryptic soy broth), saline solutions (in particular physiological saline solution), physiological solutions (such as Frog Ringer solution, Krebs' solution, Tyrode solution, Ringer-Locke solution, De Jalen solution or artificial cerebrospinal fluid) and buffer solutions.

In particular embodiments the composition is a sprayable composition. The invention thus also provides containers fitted with a spraying device such as but not limited to spray cans comprising the composition according to the invention. In particular embodiments, the composition (and/or the container) further comprises a gas propellant.

The compound of formula I according to the invention may also be encapsulated in a polymeric network or a gel-like structure such as for example described by C. Oury in WO2018/122318.

Yet a further related aspect of the invention provides a medical device, preferably a cardiovascular device, most preferably a catheter, a prosthetic heart valve or a pacemaker, coated with a compound of formula I or a composition comprising a compound of formula I as described herein. A further related aspect of the present invention provides a method ex-vivo for preventing bacterial growth in biofilm formation comprising applying or grafting on a surface an effective amount of a compound of formula I as described herein.

Bacteria in biofilms produce extracellular polymeric substances (EPS) consisting mainly of polysaccharides, nucleic acids (extracellular DNA) and proteins, that protect them from external threats, including immune system components and antimicrobials. Moreover, bacteria in a biofilm have a decreased metabolism, making them less susceptible to antibiotics; this is due to the fact that most antimicrobials require a certain degree of cellular activity in order to be effective. Another factor reinforcing such resistance is the impaired diffusion of the antimicrobial drugs throughout the biofilm because of the presence of the EPS matrix barrier.

It has been determined that the biofilm formation on any surface takes place in different states or steps. The initial step of biofilm formation is the attachment/adherence to surface, which is stronger in shear stress conditions. The protein mainly responsible for this adhesion is the polysaccharide intercellular adhesin (PIA), which allows bacteria to bind to each other, as well as to surfaces, creating the biofilm. The second stage of biofilm formation is the development of a community structure and ecosystem, which gives rise to the mature biofilm. The final stage is the detachment from the surface with consequent spreading into other locations. In all the phases of biofilm formation the quorum sensing (QS) system, mediating cell-to-cell communication, is involved.

As used herein "preventing bacterial growth in biofilm formation" refers to inhibition of biofilm formation at all stages of its formation starting from a prevention or an inhibition of adherence of bacteria on the surface at step 1 but also and mainly an inhibition in bacteria grow, multiplication, and formation of microcolonies on the surface at step 2. Inhibition of biofilm formation during the maturation step 3 and inhibition of bacteria dispersion from the matrix in a colonisation step are also considered within this definition. Bacteria may also be killed at all steps of the biofilm formation.

Accordingly, the use of the compounds of formula I according to the present invention is advantageous in inhibiting bacterial biofilm formation on surfaces.

In some embodiments the method for controlling bacterial growth in biofilm formation on a surface comprises applying or grafting on a surface an effective amount of a compound of formula I as described herein either at a prevention step, reducing bacteria adherence and survival on the substrate or at a stage where the biofilm is already present, or even at a maturation step with a matrix formation wherein a more complex architecture of biofilm is established protecting bacteria as a barrier to conventional antibacterial agent.

In some embodiments the method of prevention of bacterial growth could also be applied to the surface of an experimental device in need of such antibacterial or anti-infectious treatment.

A further related aspect of the present invention provides a compound of formula I as described herein for use in diagnosing or prognosing bacterial infection.

The compounds of formula I according to the present invention may be prepared by methods known to those skilled in the art. In particular, the synthetic methods described in WO9418216 may be used.

### Examples

The following examples are provided for the purpose of illustrating the present invention and by no means should be interpreted to limit the scope of the present invention.

### Bacteria strains and growth conditions

*Staphylococcus aureus* strains included previously characterized infective endocarditis (IE) clinical isolate (Liesenborghs L, Meyers S, Lox M, Criel M, Claes J, Peetermans M, Trenson S, Vande Velde G, Vanden Berghe P, Baatsen P, et al. Staphylococcus aureus endocarditis: distinct mechanisms of bacterial adhesion to damaged and inflamed heart valves. Eur Heart J 2019; 40:3248-3259) or JE2 (USA300 MRSA). Bacteria were grown in Tryptic soy broth (TSB, Sigma) under agitation (200 rpm) at 37°C. Prior to all experiments, a single colony from TSA plates was used to inoculate 4 mL of TSB in 15-mL polystyrene tubes and bacteria were grown overnight. Liquid cultures were then diluted 100-fold in 20 mL of fresh TSB and aliquoted in new tubes. For tetrasodium cangrelor treatment, different concentrations of the drug were added to the corresponding tubes. Tetrasodium cangrelor was dissolved in the culture medium, i.e. Tryptic soy broth which comprises proteins and nutrients for growing bacteria, more particularly: casein, soya peptone, sodium chloride, dipotassium phosphate, dextrose, water. Supernatants and bacterial pellets were collected at indicated times (exponential or stationary growth phase). Supernatants were filtered through a 0.22 µM cellulose acetate filter (VWR) and kept at -20°C until further use.

### Preparation of red blood cell suspensions and haemolytic assays

Freshly drawn citrate anticoagulated blood from healthy volunteers was centrifugated for 15 minutes at 1,000xg. After discarding plasma upper layer, red blood cells were washed once in phosphate-buffered saline (PBS) and resuspended in PBS at 2%. Supernatants from bacterial overnight culture were mixed with freshly prepared human red blood cell suspension in 1:5 final ratio and incubated for 30 minutes under gentle agitation on a table shaker (800 rpm). Samples were then centrifuged at 4,000xg for 5 minutes. Haemolytic activity was determined in supernatants by measuring the absorbance at 570 nm in a spectrophotometer.

### Analysis of staphyloxanthin production

Bacteria pellets from overnight culture were resuspended in 400 µL methanol and incubated for 30 minutes at 37°C under gentle agitation on a table shaker. Samples were centrifuged for 5 minutes at 14,000xg to remove cell debris. Pigment intensity in supernatants was analyzed by measuring the absorbance at 470 nm.

### Example 1: tetrasodium Cangrelor inhibited the haemolytic activity of Staphylococcus aureus isolated from a patient with infective endocarditis

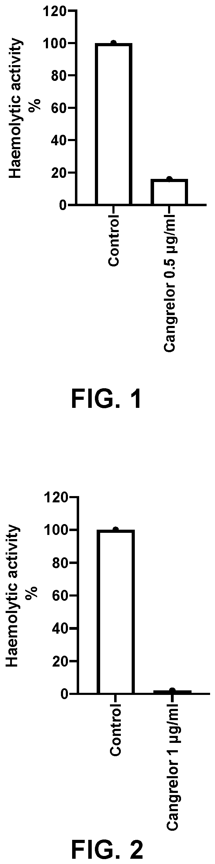

Supernatant was prepared from *Staphylococcus aureus* IE clinical isolate that was grown in the absence (Control) or in the presence of tetrasodium cangrelor (0.5µg/mL) up to exponential phase and added to red blood cell suspension for 30 minutes before assessing the haemolytic effect. Tetrasodium cangrelor was purchased from Bio-Techne Ltd. with reference AR-C 69931 tetrasodium salt. The 05µg/mL concentration of tetrasodium cangrelor corresponds to the steady state concentration of the drug reached in patient receiving conventional antiplatelet dosage, *i.e.,* intravenous bolus (30µg/kg) followed by 4-µg/kg/min infusion (Akers, W.S., Oh, J.J., Oestreich, J.H., Ferraris, S., Wethington, M. and Steinhubl, S.R. Pharmacokinetics and Pharmacodynamics of a Bolus and Infusion of Cangrelor: A Direct, Parenteral P2Y12 Receptor Antagonist. The Journal of Clinical Pharmacology 2010; 50: 27-35). Figure 1 shows that growing bacteria in the presence of tetrasodium cangrelor led to a 90% loss of supernatant haemolytic activity as compared to vehicle control (set to 100%). These data indicate that cangrelor is able to reduce the virulence of clinical *Staphylococcus aureus* strains.

### Example 2: tetrasodium Cangrelor inhibited the haemolytic activity of methicillin-resistant Staphylococcus aureus

Supernatant was prepared from the methicillin-resistant JE2 (USA300) *Staphylococcus aureus* strain that was grown in the absence (Control) or in the presence of cangrelor (1µg/mL) up to stationary phase and added to red blood cell suspension for 30 minutes before assessing the haemolytic effect. Figure 2 shows that growing bacteria in the presence of cangrelor led to a complete loss of supernatant haemolytic activity as compared to vehicle control (set to 100%). These data indicate that cangrelor is able to reduce the virulence of methicillin-resistant *Staphylococcus aureus* strains.

### Example 3: tetrasodium Cangrelor inhibited staphyloxanthin production by Staphylococcus aureus

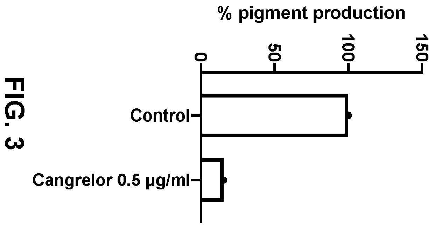

Protein extracts from *Staphylococcus aureus* IE clinical isolate that was grown in the absence (Control) or in the presence of cangrelor (0.5µg/mL) up to exponential phase were used to measure the content of the major bacteria pigment, staphyloxanthin. Figure 3 shows that growing bacteria in the presence of cangrelor led to a 90% loss of bacteria staphyloxanthin production as compared to vehicle control (set to 100%). Since staphyloxanthin has antioxidant activity that helps the bacteria evade killing by reactive oxygen species produced by phagocytic innate immune cells, these data further indicate that cangrelor is able to reduce the virulence of *Staphylococcus aureus.*

## Claims

1. A compound of formula I, a tautomer, an enantiomer or a diastereomer thereof, 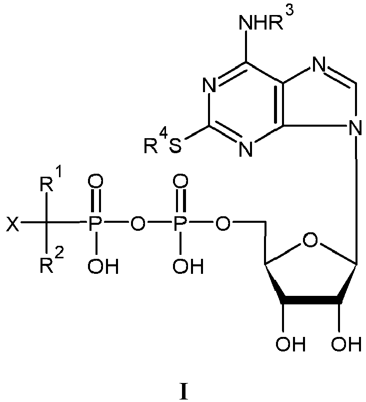 wherein,
R¹ and R² are each independently hydrogen or halogen;
R³ and R⁴ are each independently phenyl, or C₁₋₆alkyl; wherein each phenyl or C₁₋₆alkyl can be substituted or unsubstituted with one or more substituents each independently selected from the group consisting of OR⁵, C₁₋₆alkylthio, NR⁶R⁷, phenyl, COOR⁸ or halogen;
R⁵, R⁶, R⁷ and R⁸ are each independently hydrogen or C₁₋₆alkyl,
X is an acidic moiety;
or a salt or a solvate thereof or a solvate of such a salt;
for use in the prevention or treatment of a bacterial infection in a subject.

2. The compound for use according to claim 1, wherein X is selected from the group consisting of -P(O)(OH)₂, -SO₃H or -CO₂H.

3. The compound for use according to any one of claims 1 or 2, wherein R⁴ is C₁₋₆alkyl, and wherein said C₁₋₆alkyl is substituted with one, two or three substituents.

4. The compound for use according to any one of claims 1 to 3, wherein R³ is C₁₋₆alkyl, and wherein said C₁₋₆alkyl is substituted with one, two or three substituents.

5. The compound for use according to any one of claims 1 to 4, wherein R¹ and R² are the same.

6. The compound for use according to any one of claims 1 to 5, wherein the compound is a salt; preferably a sodium salt of a compound of formula I.

7. The compound for use according to any one of claims 1 to 6 which is tetrasodium cangrelor of formula III

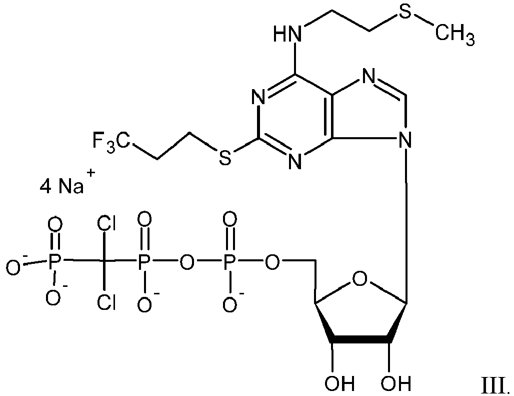

8. The compound for use according to any one of claims 1 to 7, wherein the bacterial infection is an infection caused by gram positive bacteria; preferably an infection caused by *Staphylococcus*; preferably an infection caused by *Staphylococcus aureus*; preferably an infection caused by methicillin-resistant *Staphylococcus aureus.*

9. The compound for use according to any one of claims 1 to 8, wherein said bacterial infection is an infection of a bodily surface.

10. Use ex-vivo or in vitro of a compound of formula I, a tautomer, an enantiomer or a diastereomer thereof, 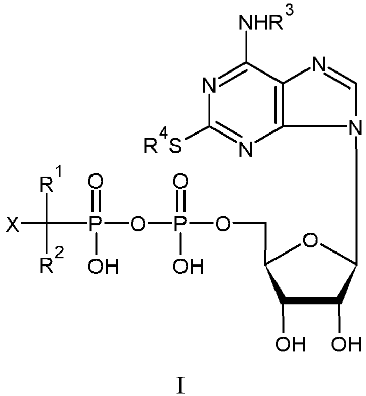 wherein,
R¹ and R² are each independently hydrogen or halogen;
R³ and R⁴ are each independently phenyl or C₁₋₆alkyl; wherein each phenyl or C₁₋₆alkyl can be substituted or unsubstituted with one or more substituents each independently selected from the group consisting of OR⁵, thioC₁₋₆alkyl, NR⁶R⁷, phenyl, COOR⁸ or halogen;
R⁵, R⁶, R⁷ and R⁸ are each independently hydrogen or C₁₋₆alkyl,
X is an acidic moiety;
or a salt or solvate thereof or a solvate of such a salt,
as an antibacterial agent.

11. Use of a compound of formula I, a tautomer, an enantiomer or a diastereomer thereof, 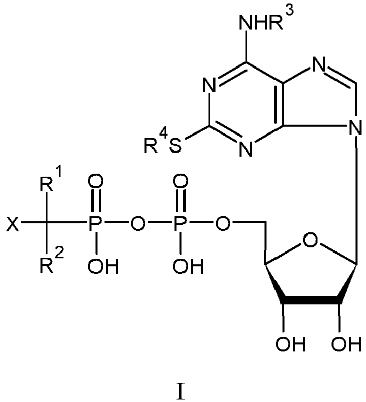 wherein,
R¹ and R² are each independently hydrogen or halogen;
R³ and R⁴ are each independently phenyl or C₁₋₆alkyl; wherein each phenyl or C₁₋₆alkyl can be substituted or unsubstituted with one or more substituents each independently selected from the group consisting of OR⁵, C₁₋₆alkylthio, NR⁶R⁷, phenyl, COOR⁸ or halogen;
R⁵, R⁶, R⁷ and R⁸ are each independently hydrogen or C₁₋₆alkyl,
X is an acidic moiety;
or a salt or solvate thereof, or a solvate of such a salt,
as inhibitor of bacterial adhesion and biofilm formation on a surface.

12. The use according to claim 11, wherein the surface is the surface of a biomaterial or a medical device; preferably the surface of a cardiovascular device or the surface of a catheter; most preferably the surface of a catheter, a prosthetic heart valve or a pacemaker.

13. A method ex-vivo for preventing bacterial growth in biofilm formation comprising applying or grafting on a surface an effective amount of a compound of formula I, a tautomer, an enantiomer or a diastereomer thereof, 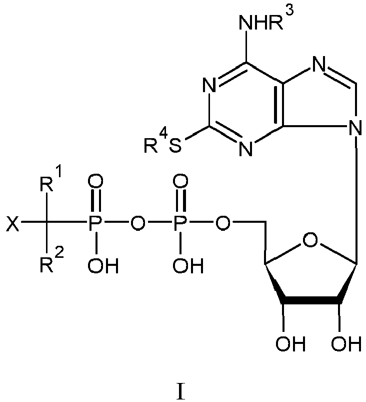 wherein,
R¹ and R² are each independently hydrogen or halogen;
R³ and R⁴ are each independently phenyl or C₁₋₆alkyl; wherein each phenyl or C₁₋₆alkyl can be substituted or unsubstituted with one or more substituents each independently selected from the group consisting of OR⁵, C₁₋₆alkylthio, NR⁶R⁷, phenyl, COOR⁸ or halogen;
R⁵, R⁶, R⁷ and R⁸ are each independently hydrogen or C₁₋₆alkyl,
X is an acidic moiety;
or a salt or a solvate thereof, or a solvate of such a salt.

14. A compound of formula I, a tautomer, an enantiomer or a diastereomer thereof, 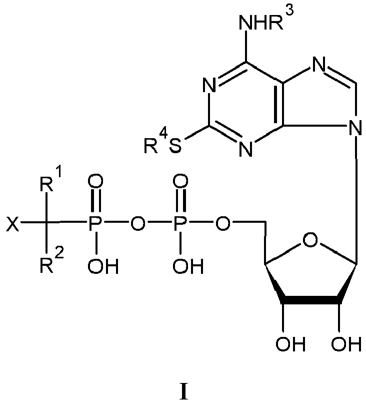 wherein,
R¹ and R² are each independently hydrogen or halogen;
R³ and R⁴ are each independently phenyl or C₁₋₆alkyl; wherein each phenyl or C₁₋₆alkyl can be substituted or unsubstituted with one or more substituents each independently selected from the group consisting of OR⁵, C₁₋₆alkylthio, NR⁶R⁷, phenyl, COOR⁸ or halogen;
R⁵, R⁶, R⁷ and R⁸ are each independently hydrogen or C₁₋₆alkyl,
X is an acidic moiety;
or a salt or a solvate thereof, or a solvate of such a salt
for use in diagnosing or prognosing bacterial infection.

15. A medical device, preferably a cardiovascular device, most preferably a catheter, a prosthetic heart valve or a pacemaker, coated with a composition comprising a compound of formula I, a tautomer, an enantiomer or a diastereomer thereof, 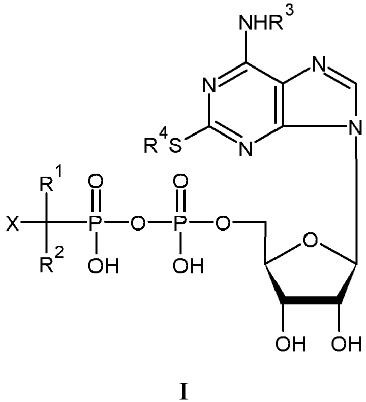 wherein,
R¹ and R² are each independently hydrogen or halogen;
R³ and R⁴ are each independently phenyl or C₁₋₆alkyl; wherein each phenyl or C₁₋₆alkyl can be substituted or unsubstituted with one or more substituents each independently selected from the group consisting of OR⁵, C₁₋₆alkylthio, NR⁶R⁷, phenyl, COOR⁸ or halogen;
R⁵, R⁶, R⁷ and R⁸ are each independently hydrogen or C₁₋₆alkyl,
X is an acidic moiety;
or a salt or a solvate thereof, or a solvate of such a salt.

16. A pharmaceutical composition comprising the compound of formula (I), a tautomer, an enantiomer or a diastereomer thereof, 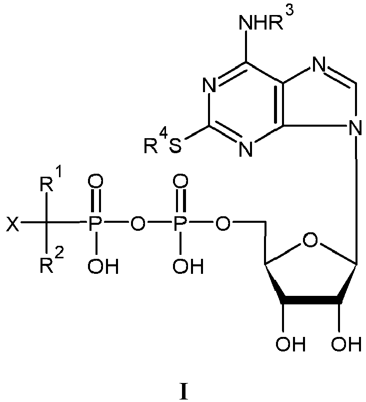 wherein,
R¹ and R² are each independently hydrogen or halogen;
R³ and R⁴ are each independently phenyl or C₁₋₆alkyl; wherein each phenyl or C₁₋₆alkyl can be substituted or unsubstituted with one or more substituents each independently selected from the group consisting of OR⁵, C₁₋₆alkylthio, NR⁶R⁷, phenyl, COOR⁸ or halogen;
R⁵, R⁶, R⁷ and R⁸ are each independently hydrogen or C₁₋₆alkyl,
X is an acidic moiety;
or a salt or a solvate thereof, or a solvate of such a salt,
for use in the prevention or treatment of a bacterial infection in a subject.

17. A composition comprising:
- a compound of formula I, a tautomer, an enantiomer or a diastereomer thereof;
- a solubilizing vehicle;
wherein the compound of formula I has the structure: 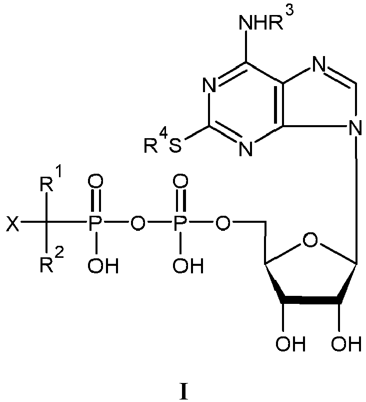 wherein,
R¹ and R² are each independently hydrogen or halogen;
R³ and R⁴ are each independently phenyl or C₁₋₆alkyl; wherein each phenyl or C₁₋₆alkyl can be substituted or unsubstituted with one or more substituents each independently selected from the group consisting of OR⁵, C₁₋₆alkylthio, NR⁶R⁷, phenyl, COOR⁸ or halogen;
R⁵, R⁶, R⁷ and R⁸ are each independently hydrogen or C₁₋₆alkyl,
X is an acidic moiety;
or a salt or a solvate thereof, or a solvate of such a salt.
